# EUROPÄISCHE PATENTSCHRIFT

(11) **EP 0 921 766 B1**
(45) Veröffentlichungstag und Bekanntmachung des Hinweises auf die Patenterteilung: **20.08.2003**
(21) Anmeldenummer: 96920680.4
(22) Anmeldetag: 09.07.1996
(51) Int. Cl.: A61B 17/70

(54) **VORRICHTUNG FÜR DIE KNOCHENCHIRURGIE**
DEVICE FOR BONE SURGERY
DISPOSITIF POUR CHIRURGIE OSSEUSE

(43) Veröffentlichungstag der Anmeldung: 16.06.1999
(73) Patentinhaber: SYNTHES AG Chur, 7002 Chur (CH)
(72) Erfinder: SCHLAEPFER, Johannes, Fridolin, CH-8750 Glarus (CH)
(74) Vertreter: Lusuardi, Werther Giovanni, Dr.
(86) Internationale Anmeldenummer: CH9600254
(87) Internationale Veröffentlichungsnummer: WO98001076

(56) Entgegenhaltungen:
- WO-A-94/17744
- DE-U- 9 314 297
- US-A- 4 289 123
- US-A- 4 773 402

## Beschreibung

Die Erfindung betrifft eine Vorrichtung für die Knochenchirurgie gemäss dem Oberbegriff des Patentanspruchs 1 und eine Fixationsvorrichtung gemäss dem Oberbegriff des Patentanspruchs 15.

Derzeit werden anteriore Fixationen im Wirbelsäulenbereich ausschliesslich mit plattenförmigen Vorrichtungen durchgeführt, wie sie beispielsweise aus der WO94/17744 bekannt sind. Solche plattenförmige Vorrichtungen haben den grossen Vorteil eine geringe Dicke (zwischen 1,1 und 2,0 mm) aufzuweisen, so dass sie nur wenig invasiv sind.
Ein Nachteil solcher plattenförmiger Vorrichtungen besteht anderseits darin, dass die Fusionszone der betroffenen Wirbelkörper nicht aktiv unter Kompression gesetzt werden kann, was unter anderem zu Pseuarthrosen führen kann. Ein weiterer Nachteil ist der notwendige, grosse Umfang des dem Chirurgen zur Verfügung stehenden Produktesortiments mit verschiedensten Dimensionen. Typischerweise sind je nach Hersteller 12 - 23 Plattentypen erforderlich.

Die Verwendung von Vorrichtungen mit Längstragern, wie sie bei der posterioren Fixation im Wirbelsäulenbereich verwendet wird, kam bisher aufgrund ihrer konstruktionsbedingten erheblichen Dicke klinisch nicht in Frage. Sie hätten jedoch den Vorteil der aktiven Kompressionsmöglichkeit und würden lediglich ein minimales Sortiment erfordern, bestehend aus zwei Backen, Längsträgern und Knochenschrauben.
Aus der US-A-4289123 ist eine Vorrichtung für die Knochenchirurgie bekannt, welche dem Oberbegriff des Anspruchs 1 entspricht. Eines der beiden zur Aufnahme einer Knochenschraube bestimmten Schraubenlöcher liegt ausserhalb des Bereiches, der durch die beiden zur Aufnahme der Längsträger bestimmten Bohrungen definiert wird.

Hier will die Erfindung Abhilfe schaffen. Der Erfindung liegt die Aufgabe zugrunde, eine Vorrichtung für die Knochenchirurgie zu schaffen, mit welcher eine anteriore Fixation im Wirbelsäulenbereich möglich ist, und welche die Vorteile der plattenförmigen Vorrichtungen (mit geringer Dicke) mit denjenigen kombiniert, welche Längsträger umfassen.
Die Erfindung löst die gestellte Aufgabe mit einer Vorrichtung, welche die Merkmale des Anspruchs 1 und einer Fixationsvorrichtung, welche die Merkmale des Anspruchs 15 aufweist.
Dank der Konkavität der auf dem Wirbelkörper aufliegenden Unterseite der plattenförmigen erfindungsgemässen Vorrichtung, tragen die lateral darin unterbringbaren Längsträger im zentralen Bereich der Vorrichtung (Bereich des Oesophagus), in welchem die Knochenschrauben eingesetzt werden, kaum zu deren Dicke bei. In diesem Bereich kann die Dicke auf 2 mm beschränkt werden.

Gleichzeitig kann die Dicke der Platte auch im Bereich der Stellschrauben, mit welchen die Längsträger fixierbar sind, dank ihrer spitzwinkligen Anordnung, gering gehalten werden (ca. 5 mm). Die Dicke der plattenförmigen Vorrichtung kann natürlich noch weiter reduziert werden, wenn Längsträger mit reduziertem Durchmesser verwendet werden.

Die Geometrie der Halswirbelkörper gestattet es, die Längsträger lateral gegenüber dem vorderen Längsband soweit posterior zu positionieren, dass die erfindungsgemässe Vorrichtung gegen das vordere Längsband hin - im Bereich des Oesophagus-eine ähnliche Dicke aufweist, wie eine konventionelle Platte. Nur im Bereich des Musculus longus colli, wo es weniger darauf ankommt, wird die Dicke der Vorrichtung durch den Durchmesser der Längsträger bestimmt.

Die erfindungsgemässe Fixationsvorrichtung besteht also lediglich aus zwei erfindungsgemäßen Vorrichtungen gemäß Anspruch 1 und zwei Längsträgern. Sie Kann ferner zwei Knochenschrauben und zwei Stellschrauben pro Backe aufweisen. Die laterale Anordnung der Längsträger trägt zusätzlich zur Rotationsstabilität und Rotationsfestigkeit der Fixationsvorrichtung bei.

Eine bevorzugte Weiterbildung der Vorrichtung besteht darin, dass der Winkel α, unter welchen die Stellschrauben zur Plattenebene der Backe verlaufen, im Bereich von 15° - 35°, vorzugsweise von 20° - 30° liegt.

Die Stellschraube weist vorzugsweise ein Gewinde mit einem Aussendurchmesser von 3 mm auf und ist derart konzipiert, dass der aus der Backe herausstehende Teil der Stellschraube bei einem definierten Anzugsmoment über eine Sollbruchstelle abgedreht wird, so dass nach erfolgter Fixation keine überstehenden Teile vorhanden sind.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine perspektivische Darstellung der erfindungsgemässen Vorrichtung mit eingeführten Längsträgern;
Fig. 2 ist eine Aufsicht auf die Vorrichtung nach Fig. 1;
Fig. 3 ist eine kombinierte Schnittdarstellung längs der Linie III-III von Fig. 2;
Fig. 4 ist eine Seitenansicht der Vorrichtung nach Fig. 1;
Fig. 5 ist eine perspektivische Darstellung der erfindungsgemässen Fixations-Vorrichtung mit 2 Vorrichtungen nach Fig. 1; und
Fig. 6 ist eine perspektivische Darstellung der erfindungsgemässen Fixations-Vorrichtung mit 2 Vorrichtungen nach Fig. 1 und einem dazwischen liegenden Wirbelkörperersatz.

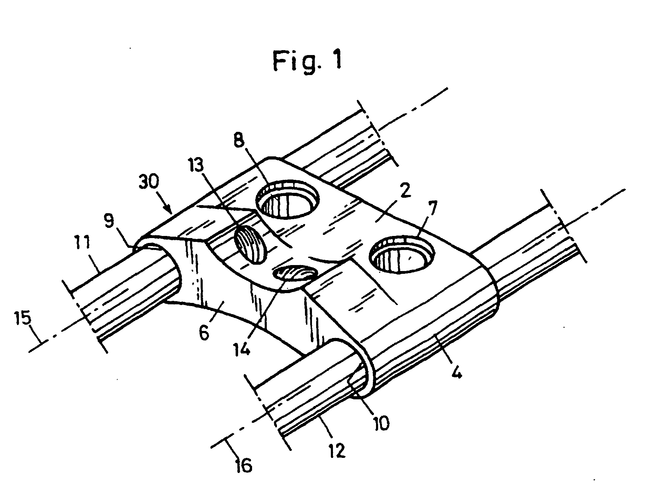

Die in den Fig. 1 - 4 dargestellte erfindungsgemässe Vorrichtung für die Knochenchirurgie besitzt im wesentlichen die Form einer Platte (im folgenden auch kurz als "Backe 30" bezeichnet) mit einer konkaven Unterseite 1, einer Oberseite 2, einer rechten Längsseite 3, einer linken Längsseite 4, einer Frontseite 5 und einer Hinterseite 6 sowie zwei zur Aufnahme von Knochenschrauben bestimmte Löcher 7,8, welche sich von der Oberseite 2 zur Unterseite 1 erstrecken und die Backe 30 durchbohren.

Die Backe 30 weist eine, zur Aufnahme eines rechten Längsträgers 11 bestimmte, rechte Bohrung 9 mit Längsachse 15 auf, welche die Frontseite 5 mit der Hinterseite 6 verbindet und benachbart zur rechten Längsseite 3 verläuft. Symmetrisch dazu weist die Backe 30 auch eine, zur Aufnahme eines linken Längsträgers 12 bestimmte, linke Bohrung 10 mit Längsachse 16 auf, welche die Frontseite 5 mit der Hinterseite 6 verbindet und benachbart zur linken Längsseite 4 verläuft.
Die beiden Bohrungen 9,10 verlaufen parallel zueinander und soweit als möglich voneinander beabstandet in den lateralen Bereichen der Backe 30.

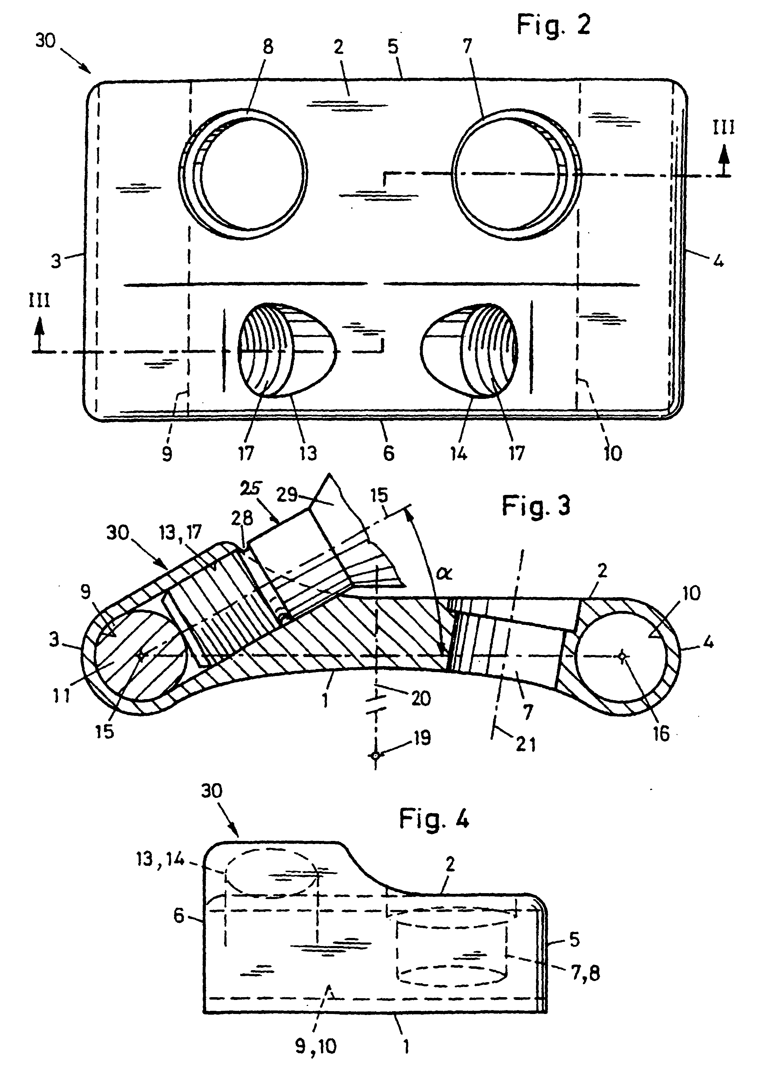

Die Backe 30 weist ferner eine, zur Aufnahme einer rechten Stellschraube 25 (Fig. 3) bestimmte, mit einem Gewinde 17 versehene rechte Öffnung 13 auf, welche die Oberseite 2 mit der rechten Bohrung 9 verbindet sowie symmetrisch dazu eine, zur Aufnahme einer linken Stellschraube bestimmte, mit einem Gewinde 17 versehene linke Öffnung 14 auf, welche die Oberseite 2 mit der linken Bohrung 10 verbindet.

Die Öffnungen 13,14 schliessen zu der von den Längsachsen 15,16 der Bohrungen 9,10 aufgespannten Ebene einen spitzen Winkel α ein, der im Bereich von 15° - 35°, vorzugsweise von 20° - 30° liegt. Die Öffnungen 13,14 konvergieren zur Oberseite 2 der Backe 30 hin, so dass sie möglichst wenig Platz beanspruchen.
Die zentrale Achse 20 (Fig. 3) der Backe 30 steht senkrecht auf der von den Längsachsen 15,16 aufgespannten Ebene.

Wie aus Fig. 3 ersichtlich bildet die Unterseite 1 ein Zylindersegment mit einem Zylinderradius zwischen 12,5 und 30 mm, vorzugsweise 20 - 27 mm, wobei die Achse 19 des Zylindersegmentes parallel zu den Längsachsen 15,16 verläuft. Vorzugsweise besteht die Unterseite 1 aus mehreren ineinander übergehenden Zylindersegmenten, wobei der Zylinderradius im Bereich der zentralen Achse 20 zwischen 20 und 30 mm und im lateralen gegen die Längsachsen 15,16 gerichteten Bereich zwischen 10 und 15 mm liegt.

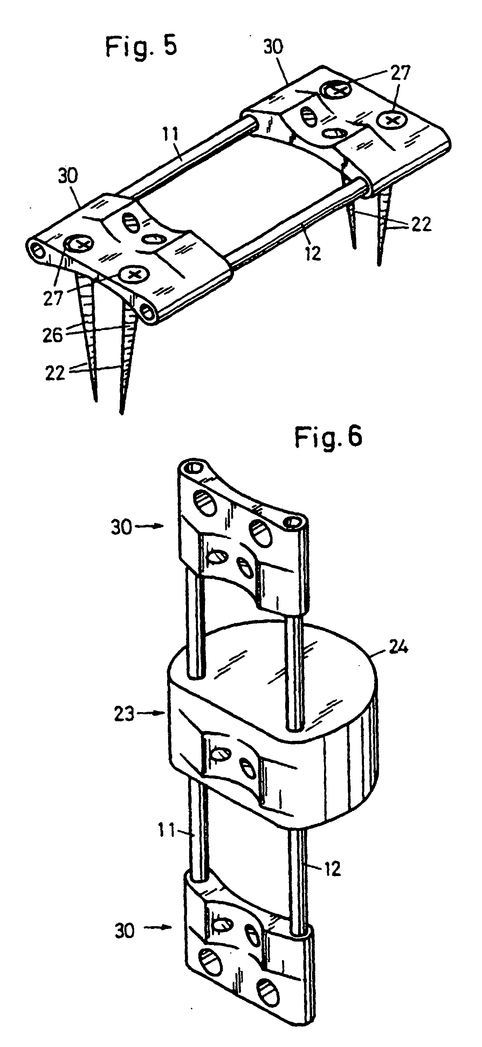

Die Löcher 7,8 weisen ein Gewinde 18 auf, welches mit Vorteil die gleiche Steigung aufweist, wie das Aussengewinde 26 der zu verwendenden Knochenschrauben 22. Es können aber auch andere Verbindungen zwischen der Backe und der Knochenschraube vorgesehen werden, z.B.:
- ein sphärisches Loch 27 (Fig. 5) zur Aufnahme einer Knochenschraube 22 mit Kugelkopf, was einen Schwenkbereich der Achse der Knochenschraube 22 zulässt;
- ein kreiszylindrisches Loch zur Aufnahme einer Knochenschraube mit Spreizkopf; oder
- ein konusförmiges Loch zu selbsthemmenden Aufnahme einer Knochenschraube mit entsprechendem Konuskopf.

In der rechten Öffnung 13 mit dem Gewinde 17 ist eine Stellschraube 25 mit einem korrespondierenden Gewinde eingeschraubt, um den rechten Längsträger 11 longitudinal und rotativ fixieren zu können. Bei einem definierten Anzugsmoment wird der Kopf 29 der Stellschraube 25 über die Sollbruchstelle 28 abgedreht, so dass nach erfolgter Fixation des rechten Längsträger 11 keine überstehenden Teile der Stellschraube 25 mehr vorhanden sind. Analog dazu kann in die linke Öffnung 14 mit dem Gewinde 17 eine Stellschraube 25 eingeschraubt werden. Der Kopf 29 der Stellschraube 25 kann mit einem Innensechskant, Torx oder Aussensechskant versehen sein.

In Fig. 5 ist eine Fixationsvorrichtung mit zwei Backen 30, welche der in den Fig. 1 - 4 beschriebenen erfindungsgemässen Vorrichtung entsprechen, zwei Längsträgern 11,12 und vier Knochenschrauben 22 dargestellt, deren Anwendung weiter unten im Detail beschrieben ist. Bei dieser Konstruktion sind die zu einer Backe 30 gehörenden Knochenschrauben 22 konvergierend ausgebildet. Die Achsen 21 (Fig. 3) der Löcher 7,8, welche in einer senkrecht zu den Längsträgern 11,12 stehenden Ebene verlaufen, weisen hierzu einen Winkel von 5° - 13°, vorzugsweise von 7° - 9° zur zentralen Achse 20 auf.
In gewissen Fällen, z.B. bei osteoporotischen Knochen ist eine divergierende Ausrichtung der Löcher 7,8 (5° - 12°, vorzugsweise 8° - 10°, von der zentralen Achse 20 aus gemessen) von Vorteil.

### Beide Anordnungen haben Vor- und Nachteile:

Bei der konvergierenden Anordnung besteht eine geringere Gefahr der Verletzung der seitlich der Wirbelkörper verlaufenden Blutgefässe.
Die divergierende Anordnung ist vorallem bei osteoporotischen Knochen von Vorteil, wegen der besseren Knochenkonsistenz gegen den Rand des Wirbelkörpers hin.

Bei gewissen Applikationen, z.B. der Verankerung im hochzervikalen oder tiefzervikalen Bereich ist es vorteilhaft, wenn die Achsen 21 der Löcher 7,8 nicht in einer senkrecht zu den Längsachsen 15,16 stehenden Ebene verlaufen, sondern einen Winkel von 5° - 15°, vorzugsweise von 10° - 12° zu einer senkrecht zu den Längsachsen 15,16 stehenden Ebene einschliessen und zur Frontseite 5 hin gerichtet sind.

In Fig. 6 ist eine Variante der erfindungsgemässen Fixationsvorrichtung nach Fig. 5 dargestellt, wie sie bei Tumorresektionen Anwendung finden kann. Bei Tumorresektionen müssen ganze wirbelkörper ersetzt werden. In solchen Situationen kann ein in die erfindungsgemässe Fixationsvorrichtung integrierter, mit den Längsträgern 11,12 verbindbarer Wirbelkörperersatz 23 verwendet werden. Dieser Wirbelkörperersatz 23 ist anterior wie die erfindungsgemässen plattenförmigen Backen 30 ausgebildet und kann somit auf beide Längsträger 11,12 geschoben werden; posterior besteht er aus einem den Zwischenwirbelraum ausfüllenden Block 24. Die gesamte Konstruktion besteht dann aus zwei Backen 30, zwei Längsträgern 11,12 und dem dazwischenliegenden Wirbelkörperersatz 23. Dank der Stabkonstruktion kann der Wirbelkörperersatz 23 unter Kompression gesetzt werden.

Im folgenden wird die Operationstechnik der erfindungsgemässen Fixationsvorrichtung im Detail beschrieben, welche folgende Schritte umfasst:
A. Auswahl der Längsträger 11,12 und der plattenförmigen Backen 30.
B. Ausrüstung der Backen 30 mit je einem Längsträger 11;12. Die Längsträger müssen dabei komplementär eingesetzt werden. Das wird dadurch erreicht, dass man die beiden Backen 30 auf einem Tisch derart anordnet, dass die Löcher 7,8 für die Knochenschrauben 22 sich (vom Betrachter weg) oben befinden. Die Längsträger 11,12 werden dann von unten in die jeweils von unten gesehen rechten Bohrungen 10 eingeführt.
C. Fixation der Längsträger 11,12 mittels der entsprechenden Stellschrauben. Die Stellschrauben werden sofort über ihrer Sollbruchstelle abgedreht. Dadurch müssen in situ nur noch zwei diagonal gegenüberliegende Stellschrauben bedient werden.
D. Zusammensetzen der Stabkonstruktion - bestehend aus je einer Backe 30 mit darin fixiertem Längsträger 11, bzw. 12 - und die verbliebenen über die Diagonale liegenden Stellschrauben leicht anziehen (ohne diese bereits abzudrehen).
E. Stabkonstruktion am Patienten einsetzen und mittels der Knochenschrauben 22 fixieren.
   Die Verbindung der beiden Backen 30 zum Knochen erfolgt mittels je zwei Knochenschrauben 22. Dabei werden die Knochenschrauben 22 je nach Ausrichtung der Löcher 7,8 entweder divergierend oder konvergierend eingebracht, um das Risiko des Ausreissens der Knochenschrauben zu minimieren.
F. Je nach Bedarf den Knochenspan, bzw. den Wirbelkörperersatz über die Stabkonstruktion komprimieren und die beiden verbliebenen Stellschrauben festziehen, indem deren Kopf über die Sollbruchstelle abgedreht wird. Dazu wird über jede Stellschraube ein abgewinkelter Ringschlüssel geschoben. Dank der in Schritt B beschriebenen speziellen Position der beiden Längsträger werden die in situ anzuziehenden Stellschrauben festgezogen, indem die Steckschlüssel von einander weg nach aussen gedreht werden. Dadurch ist es möglich, beide Stellschrauben 25 gleichzeitig anzuziehen, ohne dass sich die beiden Instrumente in die Quere kommen und das Anziehmoment wird beim Anziehen gekontert.
   Weitere Möglichkeiten, die Längsträger 11,12 zu fixieren, sind die folgenden:
   a) seitliches Klemmen der Längsträger 11.12 in den Bohrungen 9 und 10 mit Hilfe einer Zange, indem die Bohrungen 9,10 seitlich eingedrückt werden;
   b) das Klemmen wird verbessert, wenn an Stelle von Längsträgern 11,12 mit Vollprofil, Rohre mit dem gleichen Aussendurchmesser verwendet werden;
   c) Verwendung von Längsträgern 11,12 mit Aussengewinde und Muttern
   d) Aufschieben von konischen, geschlitzten Hülsen auf die Längsträger 11.12. Diese Hülsen haben einen leicht grösseren Durchmesser als die Bohrungen für die Längsträger 11,12. Durch das Einpressen der Hülsen in die Bohrungen werden die Längsträger 11,12 geklemmt.
G. In gewissen Situationen ist es erwünscht, den Knochenspan zusätzlich zu fixieren. Mit einer zusätzlichen Backe mit einem Loch in der Mitte kann auch dieses Erfordernis erfüllt werden.

## Patentansprüche

1. Vorrichtung für die Knochenchirurgie, insbesondere im zervikalen Wirbelsäulenbereich, welche im wesentlichen die Form einer plattenförmigen Backe (30) aufweist mit einer Unterseite (1), einer Oberseite (2), einer rechten Längsseite (3) einer linken Längsseite (4), einer Frontseite (5) und einer Hinterseite (6) sowie mindestens zwei zur Aufnahme von Knochenschrauben bestimmten Löchern (7,8), welche sich von der Oberseite (2) zur Unterseite (1) erstrecken und die Backe (30) durchbohren, wobei die Vorrichtung
A) eine, zur Aufnahme eines rechten Längsträgers (11) bestimmte, rechte Bohrung (9) mit Längsachse (15) aufweist, welche die Frontseite (5) mit der Hinterseite (6) verbindet und benachbart zur rechten Längsseite (3) verläuft;
B) eine, zur Aufnahme eines linken Längsträgers (12) bestimmte, linke Bohrung (10) mit Längsachse (16) aufweist, welche die Frontseite (5) mit der Hinterseite (6) verbindet und benachbart zur linken Längsseite (4) sowie parallel zur rechten Bohrung (9) verläuft; und
C) an der Unterseite (1) konkav ausgebildet ist,
**dadurch gekennzeichnet, dass**
D) die mindestens zwei zur Aufnahme von Knochenschrauben bestimmten Löcher (7,8) zwischen der rechten Bohrung (9) und der linken Bohrung (10) angeordnet sind.

2. Vorrichtung nach Anspruch 1, **dadurch gekennzeichnet, dass** sie
eine, zur Aufnahme einer rechten Stellschraube bestimmte, mit einem Gewinde (17) versehene rechte Öffnung (13) aufweist, welche die Oberseite (2) mit der rechten Bohrung (9) verbindet; und
eine, zur Aufnahme einer linken Stellschraube bestimmte, mit einem Gewinde (17) versehene linke Öffnung (14) aufweist, welche die Oberseite (2) mit der linken Bohrung (10) verbindet.

3. Vorrichtung nach Anspruch 2, **dadurch gekennzeichnet, dass** die Öffnungen (13,14) zu der von den Längsachsen (15,16) der Bohrungen (9,10) aufgespannten Ebene einen spitzen Winkel α einschliessen und zur Oberseite (2) hin konvergieren.

4. Vorrichtung nach Anspruch 3, **dadurch gekennzeichnet, dass** der Winkel α im Bereich von 15° - 35°, vorzugsweise von 20° - 30° liegt.

5. Vorrichtung nach einem der Ansprüche 1 bis 4, **dadurch gekennzeichnet, dass** die Unterseite (1) ein Zylindersegment bildet mit einem Zylinderradius zwischen 12,5 und 30 mm, vorzugsweise 20 - 27 mm, wobei die Achse (19) des Zylindersegmentes parallel zu den Längsachsen (15,16) verläuft.

6. Vorrichtung nach einem der Ansprüche 1 bis 5, **dadurch gekennzeichnet, dass** die Unterseite (1) aus mehreren ineinander übergehenden Zylindersegmenten besteht wobei der Zylinderradius im Bereich der zentralen Achse (20), welche senkrecht auf der von den Längsachsen (15,16) aufgespannten Ebene steht, zwischen 20 und 30 mm und im lateralen gegen die Längsachsen (15,16) gerichteten Bereich zwischen 10 und 15 mm liegt.

7. Vorrichtung nach einem der Ansprüche 1 bis 6, **dadurch gekennzeichnet, dass** die Achsen (21) der Löcher (7,8) in einer senkrecht zu den Längsachsen (15,16) stehenden Ebene verlaufen.

8. Vorrichtung nach einem der Ansprüche 1 bis 6, **dadurch gekennzeichnet, dass** die Achsen (21) der Löcher (7,8) einen Winkel von 5° - 15° zu einer senkrecht zu den Längsachsen (15,16) stehenden Ebene einschliessen und zur Frontseite (5) hin gerichtet sind.

9. Vorrichtung nach einem der Ansprüche 1 bis 8, **dadurch gekennzeichnet, dass** die Achsen (21) der Löcher (7,8) einen Winkel von 5° - 13°, vorzugsweise von 7° - 9° zur zentralen Achse (20), welche senkrecht auf der von den Längsachsen (15,16) aufgespannten Ebene steht, einschliessen und gegeneinander konvergieren.

10. Vorrichtung nach einem der Ansprüche 1 bis 8, **dadurch gekennzeichnet, dass** die Achsen (21) der Löcher (7,8) einen Winkel von 5° - 12°, vorzugsweise von 8° - 10° zur zentralen Achse (20), welche senkrecht auf der von den Längsachsen (15,16) aufgespannten Ebene steht, einschliessen und voneinander divergieren.

11. Vorrichtung nach einem der Ansprüche 1 bis 10, **dadurch gekennzeichnet, dass** die Löcher (7,8) ein Gewinde (18) aufweisen.

12. Vorrichtung nach einem der Ansprüche 1 bis 10, **dadurch gekennzeichnet, dass** die Löcher (7,8) im Bereich der Oberseite (2) eine sphärische Ansenkung (27) zur Aufnahme eines sphärischen Schraubenkopfes aufweisen.

13. Vorrichtung nach einem der Ansprüche 1 bis 10, **dadurch gekennzeichnet, dass** die Löcher (7,8) konisch ausgebildet sind zur Aufnahme eines konischen Schraubenkopfes.

14. Vorrichtung nach einem der Ansprüche 1 bis 10, **dadurch gekennzeichnet, dass** die Löcher (7,8) kreiszylindrisch ausgebildet sind zur Aufnahme eines kreiszylindrischen Schraubenkopfes.

15. Fixationsvorrichtung mit zwei Vorrichtungen nach einem der Ansprüche 1 bis 14, **dadurch gekennzeichnet, dass** sie zusätzlich zwei Längsträger (11,12) umfasst.

16. Fixationsvorrichtung nach Anspruch 15, **dadurch gekennzeichnet, dass** sie zusätzlich vier Stellschrauben (25) umfasst.

17. Fixationsvorrichtung nach Anspruch 15 oder 16, **dadurch gekennzeichnet, dass** sie zusätzlich vier Knochenschrauben (22) umfasst.

18. Fixationsvorrichtung nach Anspruch 11 oder 17, **dadurch gekennzeichnet, dass** die Knochenschrauben (22) ein Gewinde (26) mit der gleichen Steigung wie diejenige des Gewindes (18) der Löcher (7,8) aufweist.

19. Fixationsvorrichtung nach Anspruch 15, **dadurch gekennzeichnet, dass** die Längsträger (11,12) ein Gewinde aufweisen.

20. Fixationsvorrichtung nach Anspruch 19, **dadurch gekennzeichnet, dass** das Gewinde asymmetrisch ist.

21. Fixationsvorrichtung nach Anspruch 15, **dadurch gekennzeichnet, dass** die Längsträger (11,12) eine glatte Oberfläche aufweisen.

22. Fixationsvorrichtung nach einem der Ansprüche 16 bis 21, **dadurch gekennzeichnet, dass** der Kopf (29) der Stellschraube (25) bei einem definierten Anzugsmoment über eine Sollbruchstelle (28) abdrehbar ist.

## Claims

1. Device for bone surgery, in particular in the area of the cervical vertebrae, which essentially exhibits the shape of a plate-shaped jaw (30) with a lower surface (1), an upper surface (2), a right long side (3), a left long side (4), a front surface (5) and a rear surface (6), and also at least two holes (7, 8) intended to hold bone screws, which extend from the upper surface (2) to the lower surface (1) and bore through the jaw (30), whereby the device
A) exhibits one right bore (9) with a longitudinal axis (15) intended to hold a right longitudinal member; and this bore connects the front surface (5) with the rear surface (6) runs adjacent to the right long side (3);
B) exhibits one left bore (10) with a longitudinal axis (16) intended to hold a left longitudinal member; and this bore connects the front surface (5) with the rear surface (6) runs adjacent to the left long side (4), and also parallel to the right bore (9); and
C) has a concave shape on the lower surface,
**characterised in that**
D) the at least two holes (7, 8) intended to hold bone screws are situated between the right bore (9) and the left bore (10).

2. Device according to claim 1, **characterized in that** it
exhibits a right opening (13) provided with a thread (17), intended to hold a right fastening screw; and this opening connects the upper surface (2) with the right bore (9); and
exhibits a left opening (14) provided with a thread (17), intended to hold a left fastening screw; and this opening connects the upper surface (2) with the left bore (10).

3. Device according to claim 2, **characterized in that** the openings (13, 14) encompass an acute angle α to the plane stretched out from the longitudinal axes (15, 16) of the bores (9, 10) and converge toward the upper surface (2).

4. Device according to claim 3, **characterized in that** the angle α is in the range of 15° to 35°, preferably between 20° and 30°.

5. Device according to one of claims 1 to 4, **characterized in that** the lower surface forms a cylindrical segment with a cylindrical radius of between 12.5 and 30 mm, preferably between 20 and 27 mm, while the axis (19) of the cylindrical segment runs parallel to the longitudinal axes (15, 16).

6. Device according to one of claims 1 to 5, **characterized in that** the lower surface (1) is comprised of various cylindrical segments that merge into one another, while the cylindrical radius in the area of the central axis (2), which is located vertical on the plane stretched out from the longitudinal axes (15, 16), is located between 20 and 30 mm and between 10 and 15 mm in the lateral area directed against the longitudinal axes (15, 16).

7. Device according to one of claims 1 to 6, **characterized in that** the axes (21) of the holes (7, 8) run on a plane located vertical to the longitudinal axes (15, 16).

8. Device according to one of claims 1 to 6, **characterized in that** the axes (21) of the holes (7, 8) encompass an angle of 5° to 15° to a plane located vertical to the longitudinal axes (15, 16) and are directed toward the front surface (5).

9. Device according to one of claims 1 to 8, **characterized in that** the axes (21) of the holes (7, 8) encompass an angle of 5° to 13°, preferably between 7° and 9° to the central axis (20), which is vertical on a plane stretched out from the longitudinal axes (15, 16), and converge on one another.

10. Device according to one of claims 1 to 8, **characterized in that** the axes (21) of the holes (7, 8) encompass an angle of 5° to 12°, preferably between 8° and 10° to the central axis (20), which is vertical on a plane stretched out from the longitudinal axes (15, 16), and diverge from one another.

11. Device according to one of claims 1 to 10, **characterized in that** the holes (7, 8) exhibit a thread (18).

12. Device according to one of claims 1 to 10, **characterized in that** the holes (7, 8) exhibit a spherical counterbore (27) in the area of the upper surface (2) to hold a spherical screw head.

13. Device according to one of claims 1 to 10, **characterized in that** the holes (7, 8) are conical in shape, in order to hold a conical screw head.

14. Device according to one of claims 1 to 10, **characterized in that** the holes (7, 8) have a circular cylindrical shape to hold a circular cylindrical screw head.

15. Fixation device with two devices according to one of claims 1 to 14, **characterized in that** it also encompasses two longitudinal members (11, 12).

16. Fixation device according to claim 15, **characterized in that** it also encompasses four fastening screws (25).

17. Fixation device according to claim 15 or 16, **characterized in that** it also encompasses four bone screws (22).

18. Fixation device according to claim 11 or 17, **characterized in that** the bone screws (22) exhibit a thread (26) with the same ascending gradient as that of the thread (18) of the holes (7, 8).

19. Fixation device according to claim 15, **characterized in that** the longitudinal members (11, 12) exhibit a thread.

20. Fixation device according to claim 19, **characterized in that** the thread is asymmetrical.

21. Fixation device according to claim 15, **characterized in that** the longitudinal members (11, 12) exhibit a smooth surface.

22. Fixation device according to one of claims 16 to 21, **characterized in that** the head (29) of the fastening screw (25) can be twisted off with a defined tightening movement beyond a pre-determined breaking point (28).

## Revendications

1. Dispositif de chirurgie osseuse, en particulier pour la région cervicale de la colonne vertébrale, qui présente essentiellement la forme d'une mâchoire (30) en forme de plaque avec un côté inférieur (1), un côté supérieur (2), un côté longitudinal droit (3) et un côté longitudinal gauche (4), un côté frontal (5) et un côté arrière (6), ainsi qu'au moins deux trous (7, 8) destinés à recevoir des vis pour os, qui s'étendent depuis le côté supérieur (2) jusqu'au côté inférieur (1) et qui traversent la mâchoire (30), le dispositif présentant
A) un alésage (9) droit destiné à recevoir un support longitudinal droit (11), avec un axe longitudinal (15) qui relie le côté frontal (5) au côté arrière (6) et qui s'étend au voisinage du côté longitudinal droit (3),
B) un alésage gauche (10) destiné à recevoir un support longitudinal gauche (12), avec un axe longitudinal (16), qui relie le côté frontal (5) au côté arrière (6) et qui s'étend au voisinage du côté longitudinal gauche (4) et parallèlement à l'alésage droit (9), et
C) le dispositif est concave sur le côté inférieur (1),
**caractérisé en ce que**
D) les deux trous (7, 8) au moins présents destinés à recevoir des vis pour os sont disposés entre l'alésage droit (9) et l'alésage gauche (10).

2. Dispositif selon la revendication 1, **caractérisé en ce que**:
il présente une ouverture droite (13) dotée d'un filet (17), destinée à recevoir une vis d'ajustement droite, et qui relie le côté supérieur (2) à l'alésage de droite (9), et
une ouverture gauche (14) dotée d'un filet (17), destinée à recevoir une vis d'ajustement gauche et qui relie le côté supérieur (2) à l'alésage de gauche (10).

3. Dispositif selon la revendication 2, **caractérisé en ce que** les ouvertures (13, 14) forment par rapport au plan sous-tendu par les axes longitudinaux (15, 16) des alésages (9, 10) un angle α aigu et convergent en direction du côté supérieur (2).

4. Dispositif selon la revendication 3, **caractérisé en ce que** l'angle α est compris dans la plage de 15° à 35° et de préférence de 20° à 30°.

5. Dispositif selon l'une des revendications 1 à 4, **caractérisé en ce que** le côté inférieur (1) forme un segment de cylindre d'un rayon compris entre 12,5 et 30 mm, de préférence entre 20 et 27 mm, l'axe (19) du segment de cylindre s'étendant parallèlement aux axes longitudinaux (15, 16).

6. Dispositif selon l'une des revendications 1 à 5, **caractérisé en ce que** le côté inférieur (1) est constitué de plusieurs segments de cylindre qui se prolongent les uns dans les autres, le rayon des cylindres dans la région de l'axe central (20) qui est perpendiculaire au plan sous-tendu par les axes longitudinaux (15, 16) étant compris entre 20 et 30 mm et entre 10 et 15 mm dans la région latérale tournée vers les axes longitudinaux (15, 16).

7. Dispositif selon l'une des revendications 1 à 6, **caractérisé en ce que** les axes (21) des trous (7, 8) s'étendent dans un plan perpendiculaire aux axes longitudinaux (15, 16).

8. Dispositif selon l'une des revendications 1 à 6, **caractérisé en ce que** les axes (21) des trous (7, 8) forment un angle de 5° à 15° par rapport à un plan perpendiculaire aux axes longitudinaux (15, 16) et sont dirigés vers le côté frontal (5).

9. Dispositif selon l'une des revendications 1 à 8, **caractérisé en ce que** les axes (21) des trous (7, 8) forment un angle de 5° à 13°, de préférence de 7° à 9° par rapport à l'axe central (20) qui est perpendiculaire au plan sous-tendu par les axes longitudinaux (15, 16) et convergent l'un vers l'autre.

10. Dispositif selon l'une des revendications 1 à 8, **caractérisé en ce que** les axes (21) des trous (7, 8) forment un angle de 5° à 12°, de préférence de 8° à 10° par rapport à l'axe central (20) qui est perpendiculaire au plan sous-tendu par les axes longitudinaux (15, 16) et qui divergent l'un par rapport à l'autre.

11. Dispositif selon l'une des revendications 1 à 10, **caractérisé en ce que** les trous (7, 8) présentent un filet (18).

12. Dispositif selon l'une des revendications 1 à 10, caractérisé en ce ' que les trous (7, 8) présentent dans la région du côté supérieur (2) un creux sphérique (27) pour la réception d'une tête de vis sphérique.

13. Dispositif selon l'une des revendications 1 à 10, **caractérisé en ce que** les trous (7, 8) sont configurés en cône pour la réception d'une tête de vis conique.

14. Dispositif selon l'une des revendications 1 à 10, **caractérisé en ce que** les trous (7, 8) sont configurés en cylindre circulaire pour la réception d'une tête de vis cylindrique circulaire.

15. Dispositif de fixation doté de deux dispositifs selon l'une des revendications 1 à 14, **caractérisé en ce qu'**il comprend en outre deux supports longitudinaux (11, 12).

16. Dispositif de fixation selon la revendication 15, **caractérisé en ce qu'**il comprend en outre quatre vis d'ajustement (25).

17. Dispositif de fixation selon la revendication 15 ou 16, **caractérisé en ce qu'**il comprend en outre quatre vis pour os (22).

18. Dispositif de fixation selon la revendication 11 ou 17, **caractérisé en ce que** la vis pour os (22) présente un filet (26) du même pas que celui du filet (18) des trous (7, 8).

19. Dispositif de fixation selon la revendication 15, **caractérisé en ce que** les supports longitudinaux (11, 12) présentent un filet.

20. Dispositif de fixation selon la revendication 19, **caractérisé en ce que** le filet est asymétrique.

21. Dispositif de fixation selon la revendication 15, **caractérisé en ce que** les supports longitudinaux (11, 12) présentent une surface lisse.

22. Dispositif de fixation selon l'une des revendications 16 à 21, **caractérisé en ce que** la tête (29) de la vis d'ajustement (25) peut être serrée au-delà d'un emplacement (28) de rupture contrôlée à un couple de serrage défini.
